# EUROPEAN PATENT SPECIFICATION

(11) **EP 1 827 377 B1**
(45) Date of publication and mention of the grant of the patent: **19.03.2008**
(21) Application number: 05815330.5
(22) Date of filing: 13.12.2005
(51) Int. Cl.: A61K 9/08

(54) **IN-SITU FORMING IMPLANT FOR ANIMALS**
IN-SITU-ERZEUGUNG EINES IMPLANTATS FÜR TIERE
IMPLANT FORMÉ IN-SITU POUR LES ANIMAUX

(30) Priority: 14.12.2004 EP 04029498
(43) Date of publication of application: 05.09.2007
(73) Proprietor: Novartis AG, 4056 Basel (CH); NOVARTIS-PHARMA GMBH, 1230 Vienna (AT)
(72) Inventor: SAUTTER, Caroline, CH-4055 Basel (CH); ISELE, Ute, 79115 Freiburg (DE)
(74) Representative: Liphardt, Bernd
(86) International application number: PCT/EP2005/013377
(87) International publication number: WO 2006/063794

(56) References cited:
- EP-A- 0 539 751
- EP-A- 0 950 403
- US-A- 4 938 763
- PACKHAEUSER C B ET AL: "In situ forming parenteral drug delivery systems: an overview" EUROPEAN JOURNAL OF PHARMACEUTICS AND BIOPHARMACEUTICS, ELSEVIER SCIENCE PUBLISHERS B.V., AMSTERDAM, NL, vol. 58, no. 2, September 2004 (2004-09), pages 445-455, XP004526323 ISSN: 0939-6411
- JAIN R A: "The manufacturing techniques of various drug loaded biodegradable poly(lactide-co-glycolide) (PLGA) devices" BIOMATERIALS, ELSEVIER SCIENCE PUBLISHERS BV., BARKING, GB, vol. 21, no. 23, 1 December 2000 (2000-12-01), pages 2475-2490, XP004216917 ISSN: 0142-9612

## Description

Many attempts have been made for developing galenic formulations, applicable as slow-release carriers in drug delivery. Most often these slow-release carriers are polymers, usually made of thermoplastic resins, which liquefy or soften upon heating and re-solidify upon cooling, are generally formed into the desired structure according to their use, e. g. as surgical clips, staples or implants, prior to insertion into the body. Once inserted, they retain their shape.

Surprisingly, only few of these slow-release systems, e. g. Profact®, Zoladex® and Atridox®, reached the commercial stage seemingly indicating that the known slow-release carriers show undesired side effects hampering commercialization.

When used as drug delivery devices, the drug is incorporated within the polymeric composition and the shape of the device is formed outside the body. This solid implant is then typically inserted into the body of a human or animal through an incision. Certain polymers can be injected via a syringe as a liquid composition. Biodegradable liquid polymeric compositions applicable for slow-release drug delivery systems are described, e. g. in U. S. pat. no. 5,702,716 and U. S. pat. no. 4,938,763. These polymeric compositions are administered into the body in a liquid state or, alternatively, as a solution, typically by means of a syringe. In the body the composition coagulates or cures into a solid. One type of polymeric composition consists of a non-reactive thermoplastic polymer or co-polymer dissolved in a water-miscible solvent. This polymeric solution is placed into the body where the polymer congeals or solidifies upon dissipation or diffusion of the solvent into the surrounding body tissue.

The presence of a plasticizer within a sustained release composition is known to advance the release of bioactive material by the polymer. Known plasticizers have been used to enhance the delivery of drugs from slow-release delivery systems, e. g. described by K. Juni in Chem. Pharm. Bull. 33, 1609 (1985) and by Wong et al. in U. S. pat. no. 4,127,127. While water-insoluble liquid plasticizers are used to soften the polymer or co-polymer, increasing the diffusion coefficient for non-ionic drugs, water-soluble plasticizers are applied, if the goal is to create a microporous structure of the polymeric composition, caused by their slow leaching from the polymer resin upon exposition to an aqueous environment, thus making the composition more permeable for drugs.

Although the known liquid polymeric compositions have shown their advantages in the use for sustained drug release in medical applications, their release rates are usually slightly controllable and generally too high. Normally, minutes or hours after the implantation of the composition an undesired peak of the bioactive agent in the blood level is observed, followed by the targeted slow release over time. This peak is potentially harmful as it can lead to overdosing and toxic effects. Beyond this, the overall time for a release of an effective amount of drugs is shortened. Hence, there is a need for a liquid polymeric composition, which allows the control of the drug delivery over a defined, preferably long, period.

EP 0 950 403 describes the addition of protective carriers like vegetable oils to in-situ delivery systems to reduce the initial burst of the in-situ forming implant.

Surprisingly, the release and biodegradation properties of a polymer-based slow-release system can be significantly improved if the polymeric composition contains, besides a microporous, solid matrix of a thermoplastic polymer and a bioactive material, a large excess of a third component, which not only serves as a solvent for the thermoplastic polymer but also acts as a rate modifying agent controlling the release rate of the bioactive material. In the following this third component will nevertheless be referred to as solvent.

Hence, the present invention is, in the first instance, directed to a composition for forming a solid implant *in situ* within a body by exposure to body fluids, suitable for a controlled release of bioactive material, comprising a pharmaceutically acceptable, biodegradable, thermoplastic polymer, which is insoluble in aqueous media or human or animal body fluids, a pharmaceutically acceptable, biodegradable, water-insoluble or preferably slightly water-soluble organic solvent and a biologically active agent, characterized in that the amount by weight of said organic solvent is higher than that of said thermoplastic polymer.

Another object is to provide an accordingly improved composition for a slow-release drug delivery system that can be administered in liquid form into the body.

A further object is to provide an according composition which forms a solid matrix within the body after administration in liquid form, able to release a drug over a desired period of time.

Yet another object is to provide an according composition which allows the control of the amount and rate of the drug delivery over a preferably long term.

Hence, the invention is directed to a polymeric system, a method for therapeutic treatment using the polymeric system and the precursor of the polymeric system, a liquid composition.

The polymeric system of the present invention coagulates to a solid *in-situ* matrix upon application of the liquid composition to the aqueous medium of the body fluids, where it is essentially insoluble, while the organic solvent gradually diffuses into the surrounding body fluids. The coagulation process is responsible for the development of the rate and release control and varies as a function of the parameters and components mentioned below. Simple combination of the components without passing the stage of the liquid composition does not result in the controlled release profile of the present invention.

The essentially simultaneous diffusion and coagulation process creates the microporous structure of the matrix which is believed to be responsible for the control of the rate and extent of drug release. Under the conditions of the invention, the matrix structure exhibits a core containing large pores, enclosed by a relatively nonporous skin with very small pores. The resulting solid matrix adopts the shape of the cavity within the body into which the composition is placed.

During the process of the polymeric system coagulating to a solid *in-situ* matrix, the rate of release of the bioactive substance is generally temporarily increased until after the end of the coagulation process, which is expressed as an initial peak during the progression of the blood level of the bioactive agent after application. This may be explained by the fact that said nonporous skin, which is essentially responsible for the steady slow release of the bioactive agent, only just forms in the initial phase of the coagulation process and, thus, the rate of release is at first controlled by the porous structure of the gradually coagulating core of the liquid polymeric system immediately after its application into the body. This temporary peak in the release rate can cause a momentary overdose of the bioactive compound, which is undesired and in some cases might be harmful. The amount in height and time of the initial peak release rate is a function of the concentration of the bioactive material in the polymeric system, the viscosity of the latter and the concentration and kind of adjuvants at the time of application.

It has been surprisingly found out that substitution of up to 10% of the solvent by an organic, water-soluble adjuvant to the liquid composition has a decidedly decreasing effect on the initial peak of the blood level of the bioactive agent immediately after application.

It is therefore another object of the present invention to provide a composition which effectively suppresses the initial peak release rate after the application of the liquid polymeric system into the body of a human or animal in that said composition additionally encompasses an organic, water-soluble adjuvant.

The long-term *in-situ* rate and extent of the drug release of the matrix - i. e. after the initial peak release rate - can be controlled by the variation of the parameters and conditions of the invention. This control can be accomplished by
a) variation of polymer type and molecular weight,
b) the concentration of the polymer,
c) the water-solubility properties of the organic solvent,
d) the concentration of the organic solvent,
e) the concentration of the bioactive material,
f) the form of the bioactive material,
g) further adjuvants and
h) the concentration of the adjuvants present within the matrix.

Preferably, within the scope of the invention the rate and extent of the drug release is controlled by the variation of the organic solvent and its concentration, the concentration of the bioactive material, the presence or absence of further adjuvants and their concentration.

More preferably, the rate and extent of the drug release is controlled by the variation of the organic solvent and the presence or absence of further adjuvants.

Most preferably, the rate and extent of the drug release is controlled by the variation of the parameters and conditions given in the examples below.

The method of the invention is based upon the blood level measurement of the *in-situ* controlled release of the bioactive material from the polymer system. The implantation of the liquid composition can generally occur anywhere within the body of a human or animal. Examples include soft tissue such as muscle or fat, or the subcutaneous tissue. The liquid composition can be administered by any suitable method, as for example by means of a syringe needle.

The polymer system is prepared by combining the liquid composition and an aqueous medium, such as the body fluids, to coagulate the composition into a solid, microporous, polymeric matrix. The liquid composition contains a biocompatible, thermoplastic polymer or co-polymer in combination with a biocompatible, organic solvent and optionally a biocompatible adjuvant. The thermoplastic polymer or co-polymer is biodegradable and/or bioerodible within the body of the human or animal. The biodegradability enables the body to metabolize the polymer matrix allowing it to excrete it without the need for surgery to remove it. Selection of biocompatible material makes sure that the insertion process and the presence of the polymer system within the body do not cause substantial tissue irritation or necrosis at the site of the implant.

Suitable thermoplastic polymers or co-polymers for the incorporation as the solid matrix of the controlled release system include polylactides, polyglycolides, polycaprolactones, polyanhydrides, polyamides, polyurethanes, polyester amides, polyorthoesters, polydioxanones, polyacetals, polyketals, polycarbonates, polyorthocarbonates, polyphosphazenes, polyhydroxybutyrates, polyhydroxyvalerates, polyalkylene oxalates, polyalkylene succinates, poly(malic acid) polymers, polymaleic anhydrides, poly(methylvinyl) ethers, poly(amino acids), chitin, chitosan, and copolymers, terpolymers, or combinations or mixtures of the above materials.

Preferred materials are polylactides, in particular polylactic acid, glycolic acid and copolymers thereof, most particularly polylactic acid. These polymers show excellent biocompatibility, as they produce little, if any, tissue irritation, inflammation, necrosis, or toxicity. In the presence of water, these polymers degrade to lactic and glycolic acid, respectively, which are readily metabolized in the body.

The concentration of the thermoplastic polymer in the liquid composition lies in the range of about 10% to about 25%, preferably in the range of about 15% to about 20% of the total weight of the composition.

According to the practice of the invention, the liquid composition containing the thermoplastic polymer, organic solvent, bioactive material and potentially adjuvant is a stable liquid substance. Depending on the bioactive material and solvent chosen, either a homogeneous solution or a suspension or dispersion of the bioactive material in the liquid composition results. In either case, the thermoplastic polymer is substantially soluble in the liquid composition. Upon placement of the liquid composition into the aqueous medium inside the body, the polymer will solidify to form the polymer system carrying within a solid matrix the bioactive material and a decreasing amount of the gradually diffusing organic solvent, which acts as rate-modifying agent.

The adjuvants optionally used in the thermoplastic compositions of the invention are preferably pharmaceutically acceptable, water-miscible and biocompatible. Preferably, they cause relatively little, if any, tissue irritation or necrosis at the site of the injection. The solvent is water-miscible to allow it to quickly dissipate from the polymeric composition into the aqueous body fluids, concomitantly accelerating the formation of the nonporous skin and hence suppressing the initial peak release rate of the drug after the application.

Examples of suitable adjuvants encompass N-methyl-2-pyrrolidone, 2-pyrrolidone, C₂-C₆alkanols 2-ethoxyethanol, polyhydroxy alcohols such as propylene glycol, polyethylene glycol, glycerol or sorbitol, alkyl esters such as 2-ethoxyethyl acetate, methyl acetate, ethyl acetate, propylene carbonate or ethyl lactate, ethylene glycol dimethyl ether, propylene glycol, alkyl ketones such as acetone or methyl ethyl ketone, ketals such as glycerol formal, dimethylformamide, dimethyl sulfoxide, dimethyl sulfone, tetrahydrofuran, and cyclic alkyl amides such as caprolactam. The preferred adjuvants are C₂-C₆alkanols, polyhydroxy alcohols or ketals, in particular ethanol, glycerol or glycerol formal.

The water-soluble or slightly water-soluble, organic solvents used in the present invention are known to control the sustained release character of the polymer system. The combination of a suitable solvent and a matrix formed by an aggregation process as outlined above has a significantly retarding effect on the release rate of the bioactive material in comparison with a matrix without a corresponding solvent. The retarding effect can lie in the range of several orders of magnitude, depending on the structure and amount of the rate-modifying solvent. Thus, by the appropriate choice of the thermoplastic polymer or co-polymer, combined with a suitable solvent, the rate and extent of release of bioactive material from the polymer system can deliberately be varied from very fast to very slow.

Solvents acting as rate modifying agents in the present invention are liquids which dissolve the thermoplastic polymer and are preferably water-insoluble or slightly water-soluble. They also preferably have a high boiling point. The ideal rate-modifying solvent imparts to the finally coagulated polymer matrix a glass transition temperature at about the temperature of the human or animal body or below, ensuring a soft, resilient and flexible implant.

The solvents used in the present invention are pharmaceutically acceptable. Specific examples encompass esters of mono-, di- and tricarboxylic acids such as 2-ethoxyethyl acetate, methyl acetate, ethyl acetate, diethyl phthalate, dimethyl phthalate, dibutyl phthalate, dimethyl adipate, dimethyl succinate, dimethyl oxalate, dimethyl citrate, triethyl citrate, acetyl tributyl citrate, acetyl triethyl citrate or di(n-butyl) sebacate; fatty acids; triesters of glycerol such as glycerol triacetate (triacetin), epoxidized soybean oil and other epoxidized vegetable oils; sterols such as cholesterol; alcohols such as C₆-C₁₂ alkanols; and mixtures thereof. Preferred solvents are triesters of glycerol, in particular glycerol triacetate (triacetin).

The amount of the rate modifying solvent in the thermoplastic composition preferably exceeds that of the polymer, more preferably it lies in the range of about 50% to about 80%, most preferably in the range of about 55% to about 66% of the total weight of the composition.

The term "drug", "bioactive material" or "biologically active agent" as used herein encompasses biologically, physiologically or pharmacologically active substances that act locally or systemically in the human or animal body. The bioactive material can be applied in various forms which are capable of being released from the polymer system into the adjacent body tissues or fluids. Due to the high amount of organic, rate-modifying solvent in the thermoplastic composition of this invention the bioactive material need not be water-soluble as it is gradually carried into the environmental tissue by the slightly water-soluble solvent in a dissolved or micro-dispersed manner.

Generally, any bioactive material can be applied in the liquid composition of the present invention. Representative bioactive materials applicable in the injectable sustained release compositions of the present invention encompass human as well as animal drugs, such as peptide drugs, protein drugs, desensitizing agents, antigens, vaccines, anti-infectives, antibiotics, antimicrobials, antiallergenics, steroidal anti-inflammatory agents, decongestants, miotics, anticholinergics, sympathomimetics, sedatives, hypnotics, psychic energizers, tranquilizers, androgenic steroids, estrogens, progestational agents, humoral agents, prostaglandins, analgesics, antispasmodics, antimalarials, antihistamines, cardioactive agents, non-steroidal anti-inflammatory agents, antiparkinsonian agents, antihypertensive agents, β-adrenergic blocking agents, nutritional agents, benzophenanthridine alkaloids, acaricides and insecticides. To those skilled in the art, other drugs or bioactive materials that can be released in an aqueous environment can be utilized in the described injectable system. Also, various forms of the drugs or bioactive materials may be used. These include without limitation forms such as uncharged molecules, molecular complexes salts, ethers , esters, amides, etc., which are biologically activated when injected into the body.

The bioactive material can be miscible in the polymer, organic solvent and/or adjuvant to provide a homogeneous mixture with the polymer, or insoluble in the polymer, organic solvent and/or adjuvant to form a suspension or dispersion in the composition. Preferably, the bioactive material is dissolved in the liquid composition.

The polymer system is formulated in a manner as to contain the bioactive material in an amount effective to provide the desired biological, physiological and/or therapeutic effect. The "effective amount" of a bioactive material incorporated into the polymeric composition depends on various factors, such as the desired release profile, the concentration of bioactive material required for a desired biological effect, and the period of time over which the bioactive material needs to be released for a specific treatment. Ultimately, this amount is determined by the human or animal patient's physician or veterinarian, respectively, who will apply his experience and knowledge in prescribing the appropriate amount of bioactive material for a successful treatment. Generally, the critical upper limit on the amount of bioactive material incorporated into the polymer composition is defined by the maximum peak of the initial burst release, which could cause toxic side effects, and by the need of an acceptable solution or dispersion viscosity for injection through a syringe needle. The lower limit of drug incorporated into the delivery system is only dependent on the activity of the drug and the length of time needed for treatment.

When the liquid composition is injected into soft tissue to provide a sustained release implant, the resulting polymer system will both release the bioactive material and biodegrade as designed so that no residue remains. With certain drugs, the polymer system will degrade after the drug has been completely released. In other cases, the drug will be released only after the polymer system has degraded to a point where the retained drug has been exposed to the body fluids.

The following examples are set forth as representative specific and preferred embodiments of the present invention. These examples are not to be construed as limiting the scope of the invention in any manner. It should be understood that many variations and modifications can be made while remaining within the spirit and scope of the invention. The bioactive ingredient used in the examples is a mixture of two compounds of formula 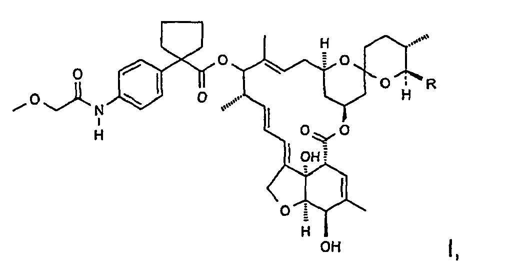 where R is hydrogen and methyl, respectively, in a 1 : 4 - proportion.

### Example 1: Preparation of formulations

Under aseptic conditions, a quantity of the thermoplastic polymer and the organic solvent is weighed into a beaker and stirred at 300 rpm for about 12 hours at about 60°C until the polymer is completely dissolved. The mixture is then cooled to room temperature, the bioactive agent added and the suspension gently stirred at 100 rpm until the bioactive agent is completely dissolved. If additionally a water-soluble adjuvant is used, the bioactive agent is first triturated in the adjuvant and then added. The final formulation is then filled into a syringe of 2 ml content. The composition of six test formulations are tabulated in table 1.

**Table 1: Composition [mg/ml] of sustained-release injectable formulations**

| Formulation | Bioactive ingredient ¹ | Thermoplastic polymer² | Solvent³ | Adjuvant: Ethanol | Adjuvant: Glycerol formal | Adjuvant: Glycerol |
|---|---|---|---|---|---|---|
| A | 200 | 200 | 799 | - | - | - |
| B | 200 | 150 | 845 | - | - | - |
| C | 200 | 200 | 653 | 100 | - | - |
| D | 200 | 150 | 698 | 100 | - | - |
| E | 200 | 200 | 702 | - | 100 | - |
| F | 200 | 200 | 705 | - | - | 100 |

| | | | | | | |
|---|---|---|---|---|---|---|
| ¹ Compound of formula I ² Polylactic acid (PLA) ³ Glycerol triacetate (triacetin) | | | | | | |

### Example 2: in-vivo Experiments on Beagle dogs

The following *in-vivo* experiments are carried out on six pairs of healthy Beagle dogs of various age, breed, body weight and sex. Each formulation is tested in two dogs, each dog receiving one injection with the active agent and a corresponding placebo, wherein the active agent is replaced by an additional amount of the organic solvent. The active-agent formulations are administered subcutaneously behind the shoulder and over the ribs of the left side. The same volume of placebo solution is injected subcutaneously on the right side of the same animal. The total injection volume per formulation is 2 ml. Consequently, the total amount of active agent per dog is 400 mg, corresponding to a dose of about 40 to about 44 mg/kg of active agent, depending on the body weight of the selected dog.

Blood samples are collected from the *vena jugularis* of each dog into sterile tubes of about 2.7 ml volume containing EDTA as anticoagulant. Blood is collected at pre-test time and at 2 h, 4 h, 6 h, 8 h, 10 h and 24 h, then at day 2, 4, 7, 10, 14, 17, 21, 24, 28, 31, 35, 38, 42 and 45, finally continued biweekly until up to 462 days after administration of the test formulations. The blood samples are kept frozen until LC-MS analysis.

In table 2 the blood levels over time for the formulations A - F are listed. The third column of this table shows that addition of the adjuvants ethanol, glycerol formal or glycerol (formulations C, D, E and F) decreases the maximum blood level reached a few days after injection.

**Table 2: Blood level [ng/ml] of compound of formula I in adult Beagle dogs after injection**

| **Formulation** | **Dog no.** | **Maximum (days after injection)** | **50 days** | **100 days** | **200 days** | **400 days** |
|---|---|---|---|---|---|---|
| A | 1 | 89.2 (2) | 8.2 | 7.6 | 3.5 | 4.1 |
| A | 2 | 87.6 (2) | 44.7 | 32.1 | 5.7 | 5.9 |
| B | 3 | 92.7 (2) | 27.4 | 26.2 | 26.7 | 5.6 |
| B | 4 | 117.2 (13) | 21 | 15.3 | 7.4 | 8.9 |
| C | 5 | 44.2 (3) | 2.4 | 3.3 | 9.8 | 14 |
| C | 6 | 47.5 (4) | 5.4 | 4.2 | 2.6 | 1.9 |
| D | 7 | 61.2 (1) | 7.1 | 3.8 | 2.1 | - |
| D | 8 | 48.9 (2) | 10.1 | 4.8 | 3.1 | - |
| E | 9 | 35.6 (2) | 4.9 | 2.8 | 1.9 | 3.0 |
| E | 10 | 37.4 (6) | 11.5 | 7.4 | 3.7 | 3.4 |
| F | 11 | 35.0 (2) | 3.3 | 2.6 | 1.0 | 1.8 |
| F | 12 | 40.6 (2) | 2.2 | 2.5 | 2.0 | 2.4 |

## Claims

1. A composition for forming a solid implant *in situ* within a body by exposure to body fluids, suitable for a controlled release of bioactive material, comprising a pharmaceutically acceptable, biodegradable, thermoplastic polymer, which is insoluble in aqueous media or human or animal body fluids, a pharmaceutically acceptable, biodegradable, water-insoluble or slightly water-soluble organic solvent, a pharmaceutically acceptable, water-soluble and biocompatible adjuvant and a biologically active agent, **characterized in that** the amount by weight of said solvent is higher than that of said thermoplastic polymer and **in that** the adjuvant is selected from the group consisting of ethanol, glycerol and glycerol formal.

2. A composition according to claim 1, **characterized in that** the amount of said thermoplastic polymer is between about 10% and about 25% and the amount of said solvent is between about 50% and about 80% by weight of the composition.

3. A composition according to claim 1, **characterized in that** the amount of said thermoplastic polymer is between about 15% and about 20% and the amount of said solvent is between about 55% and about 66% by weight of the composition.

4. A composition according to any one of claims 1 to 3, **characterized in that** the thermoplastic polymer is a polylactide.

5. A composition according to any one of claims 1 to 3, **characterized in that** the thermoplastic polymer is polylactic acid.

6. A composition according to any one of claims 1 to 3, **characterized in that** the solvent is a triester of glycerol.

7. A composition according to any one of claims 1 to 3, **characterized in that** the solvent is glycerol triacetate.

8. A composition according to claim 1, **characterized in that** the adjuvant is selected from the group consisting of C₂-C₆alkanols, polyhydroxy alcohols and ketals.

9. A composition according to claim 1, **characterized in that** the adjuvant is glycerol.

10. A composition according to any one of claims 1 to 9, **characterized in that** the biologically active agent is an animal drug.

11. A composition according to claim 10, **characterized in that** the biologically active agent is an acaricide or insecticide.

12. A composition according to claim 11, **characterized in that** the biologically active agent is a compound of formula 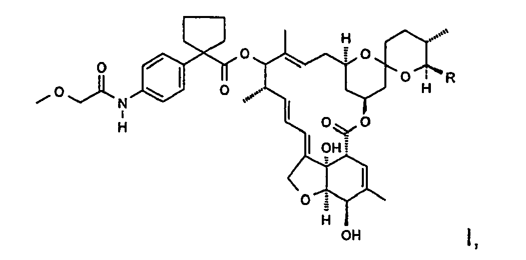 where R is hydrogen and methyl, respectively, in a 1 : 4 - proportion.

13. A composition according to any one of claims 1 to 3, **characterized in that** the thermoplastic polymer is a polylactide, the solvent is a triester of glycerol, and the biologically active agent is an animal drug.

14. A composition according to any one of claims 1 to 3, **characterized in that** the thermoplastic polymer is polylactic acid, the solvent is glycerol triacetate, and the biologically active agent is an acaricide or insecticide.

15. A composition according to claim 14, **characterized in that** the adjuvant is ethanol.

16. A composition according to claim 14, **characterized in that** the biologically active agent is a compound of formula I according to claim 12.

17. A composition according to claim 16, **characterized in that** the adjuvant is ethanol.

18. A composition according to any one of claims 1 to 17 for forming an implant for the slow release of drugs in a human or animal body.

19. An implant for the slow release of drugs in a human or animal body, **characterized in that** said implant is formed *in situ* within said body by exposure of a composition according to any one of claims 1 to 17 to the fluids of said body.

20. A composition according to any one of claims 1 to 17 for use in a method of implanting said composition into a human or animal body, **characterized in that** said composition is injected into the tissue of said body, therein forming a solid implant on contact with said body's fluids.

## Patentansprüche

1. Zusammensetzung zum Bilden eines festen Implantats in situ in einem Körper, indem sie Körperflüssigkeiten ausgesetzt wird, die für die kontrollierte Freisetzung von biologisch aktivem Material geeignet sind, umfassend ein pharmazeutisch akzeptables, biologisch abbaubares, thermoplastisches Polymer, das in wässerigen Medien oder menschlichen oder tierischen Körperflüssigkeiten unlöslich ist, ein pharmazeutisch akzeptables, biologisch abbaubares, wasserunlösliches oder schwach wasserlösliches organisches Lösungsmittel, ein pharmazeutisch akzeptables, wasserlösliches und biokompatibles Adjuvans und ein biologisch aktives Mittel, **dadurch gekennzeichnet, daß** die Menge, bezogen auf das Gewicht, des Lösungsmittels höher als die des thermoplastischen Polymers ist und daß das Adjuvans aus der Gruppe, bestehend aus Ethanol, Glycerol und Glycerolformal, ausgewählt ist.

2. Zusammensetzung nach Anspruch 1, **dadurch gekennzeichnet, daß** die Menge des thermoplastischen Polymers zwischen etwa 10 % und etwa 25 % und die Menge des Lösungsmittels zwischen etwa 50 % und etwa 80 %, bezogen auf das Gewicht der Zusammensetzung, liegt.

3. Zusammensetzung nach Anspruch 1, **dadurch gekennzeichnet, daß** die Menge des thermoplastischen Polymers zwischen etwa 15 % und etwa 20 % und die Menge des Lösungsmittels zwischen etwa 55 % und etwa 66 %, bezogen auf das Gewicht der Zusammensetzung, liegt.

4. Zusammensetzung nach einem der Ansprüche 1 bis 3, **dadurch gekennzeichnet, daß** das thermoplastische Polymer ein Polylactid ist.

5. Zusammensetzung nach einem der Ansprüche 1 bis 3, **dadurch gekennzeichnet, daß** das thermoplastische Polymer Polymilchsäure ist.

6. Zusammensetzung nach einem der Ansprüche 1 bis 3, **dadurch gekennzeichnet, daß** das Lösungsmittel ein Triester von Glycerol ist.

7. Zusammensetzung nach einem der Ansprüche 1 bis 3, **dadurch gekennzeichnet, daß** das Lösungsmittel Glyceroltriacetat ist.

8. Zusammensetzung nach Anspruch 1, **dadurch gekennzeichnet, daß** das Adjuvans aus der Gruppe, bestehend aus C₂-C₆-Alkanolen, Polyhydroxyalkoholen und Ketalen, ausgewählt ist.

9. Zusammensetzung nach Anspruch 1, **dadurch gekennzeichnet, daß** das Adjuvans Glycerol ist.

10. Zusammensetzung nach einem der Ansprüche 1 bis 9, **dadurch gekennzeichnet, daß** das biologisch aktive Mittel ein Arzneimittel für Tiere ist.

11. Zusammensetzung nach Anspruch 10, **dadurch gekennzeichnet, daß** das biologisch aktive Mittel ein Akarizid oder Insektizid ist.

12. Zusammensetzung nach Anspruch 11, **dadurch gekennzeichnet daß** das biologisch aktive Mittel eine Verbindung der Formel 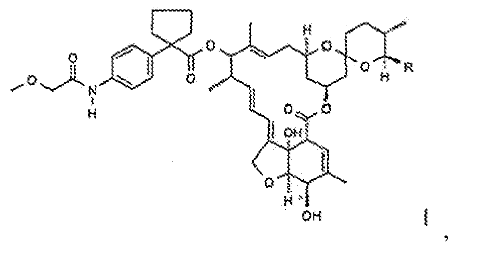 worin R Wasserstoff bzw. Methyl ist, in einem 1 : 4-Anteil ist.

13. Zusammensetzung nach einem der Ansprüche 1 bis 3, **dadurch gekennzeichnet, daß** das thermoplastische Polymer ein Polylactid ist, das Lösungsmittel ein Triester von Glycerol ist und das biologisch aktive Mittel ein Arzneimittel für Tiere ist.

14. Zusammensetzung nach einem der Ansprüche 1 bis 3, **dadurch gekennzeichnet, daß** das thermoplastische Polymer Polymilchsäure ist, das Lösungsmittel Glyceroltriacetat ist und das biologisch aktive Mittel ein Akarizid oder Insektizid ist.

15. Zusammensetzung nach Anspruch 14, **dadurch gekennzeichnet, daß** das Adjuvans Ethanol ist.

16. Zusammensetzung nach Anspruch 14, **dadurch gekennzeichnet, daß** das biologisch aktive Mittel eine Verbindung der Formel I nach Anspruch 12 ist.

17. Zusammensetzung nach Anspruch 16, **dadurch gekennzeichnet, daß** das Adjuvans Ethanol ist.

18. Zusammensetzung nach einem der Ansprüche 1 bis 17 zur Bildung eines Implantats für die langsame Freisetzung von Arzneimitteln im Körper eines Menschen oder eines Tiers.

19. Implantat für die langsame Freisetzung von Arzneimitteln im Körper eines Menschen oder eines Tiers, **dadurch gekennzeichnet, daß** das Implantat in situ im Körper gebildet wird, indem eine Zusammensetzung nach einem der Ansprüche 1 bis 17 den Flüssigkeiten dieses Körpers ausgesetzt wird.

20. Zusammensetzung nach einem der Ansprüche 1 bis 17 zur Verwendung in einem Verfahren zum Implantieren der Zusammensetzung in den Körper eines Menschen oder eines Tiers, **dadurch gekennzeichnet, daß** die Zusammensetzung in das Körpergewebe injiziert wird und darin beim Kontakt mit den Körperflüssigkeiten ein festes Implantat bildet.

## Revendications

1. Composition destinée à former un implant solide *in situ* dans un corps par exposition aux fluides corporels, convenant pour la libération contrôlée d'un matériau bioactif, comprenant un polymère thermoplastique, biodégradable et pharmaceutiquement acceptable, insoluble dans un milieu aqueux ou dans les fluides corporels humains ou animaliers, un solvant organique insoluble ou légèrement soluble dans l'eau, biodégradable et pharmaceutiquement acceptable,un adjuvant hydrosoluble et biocompatible pharmaceutiquement acceptable et un agent biologiquement actif, **caractérisée en ce que** la quantité en poids dudit solvant est supérieure à celle dudit polymère thermoplastique et **en ce que** l'adjuvant est sélectionné à partir du groupe composé d'éthanol, de glycérol et formal de glycérol,

2. Composition selon la revendication 1, **caractérisée, en ce que** la quantité dudit polymère thermoplastique est comprise dans la gamme allant d'environ 10% et environ 25% et **en ce que** la quantité dudit solvant est comprise dans la gamme allant d'environ 50% et environ 80% en poids de la composition.

3. Composition selon la revendication 1 , **caractérisée en ce que** la quantité dudit polymère thermoplastique est comprise dans la gamme allant d'environ 15% et environ 20% et **en ce que** la quantité dudit solvant est comprise dans la gamme allant d'environ 55% et environ 66% en poids de la composition.

4. Composition selon l'une quelconque des revendications 1 à 3, **caractérisée en ce que** le polymère thermoplastique est un polulactide.

5. Composition selon l'une quelconque des revendications 1 à 3, **caractérisée en ce, que** le polymère thermoplastique est un acide polyacetique.

6. Composition selon l'une quelconque des revendications 1 à 3 , **caractérisée en ce que** le solvant est un tri-ester de glycérol.

7. Composition selon l'une quelconque des revendications 1 à 3, **caractérisée en ce que** le solvant est le tri-acétate de glycérol.

8. Composition selon la revendication 1, **caractérisée en ce que** l'adjuvant est sélectionné à partir du groupe composé de C₂-C₆alkanols, alcohols polyhydroxy et cétals.

9. Composition selon la revendication 1, **caractérisée en ce que** l'adjuvant est du glycérol.

10. Composition selon l'une quelconque des revendications 1 à 9, **caractérisée en ce que** l'agent biologiquement actif est un produit animalier.

11. Composition selon la revendication 10, **caractérisée en ce que** l'agent biologiquement actif est un acaricide ou un insecticide.

12. Composition selon la revendication 11, **caractérisée en ce que** l'agent biologiquement actif est un composé de formule 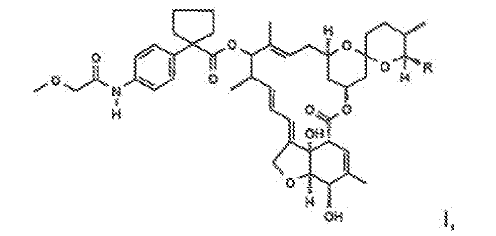 dans laquelle, R est hydrogène et méthyle, respectivement selon une proportion 1:4.

13. Composition selon l'une quelconque des revendication 1 à 3 , **caractérisée en ce que** le polymère thermoplastique est un polylactide, **en ce que** le solvant est un tri-ester de glycérol, et **en ce que** l'agent biologiquement actif est un produit animalier.

14. Composition selon l'une quelconque des 1 à 3, **caractérisée en ce que** le polymère thermoplastique est un acide polylactique, **en ce que** le solvant est un tri-acétate de glycérol, et **en ce que** l'agent biologiquement actif est un acaricide ou un insecticide.

15. Composition selon la revendication 14, **caractérisée en ce que** l'adjuvant est de l'éthanol.

16. Composition selon la revendication 14, **caractérisée en ce que** l'agent biologiquement actif est un composé de formule I selon la revendication 12.

17. Composition selon la revendication 16, **caractérisée en ce que** l'adjuvant est de l'ethanol.

18. Composition selon l'une quelconque des revendications 1 à 17 destinée à former un implant en vue de la libération lente de produits dans un corps humain ou animal.

19. Implant pour la libération lente de produits dans un corps humain ou animal, **caractérisé en ce que** ledit implant est formé *in situ* dans ledit corps par exposition d'une composition selon l'une quelconque des revendications 1 à 17 aux fluides dudit corps.

20. Composition selon l'une quelconque des revendications 1 à 17 destinée à être employée dans une méthode d'implantation de ladite composition dans un corps humain ou animal, **caractérisé en ce que** ladite est injectée dans les tissus dudit corps où elle forme un implant solide qui est avec lesdits fluides corporels,
